# EUROPEAN PATENT SPECIFICATION

(11) **EP 4 449 984 B1**
(45) Date of publication and mention of the grant of the patent: **19.08.2026**
(21) Application number: 22924856.2
(22) Date of filing: 07.02.2022
(51) Int. Cl.: A61B 5/08, G01N 21/3504, G01N 21/53, G01N 15/0205, G01N 21/31, G01N 33/497

(54) **RESPIRATORY SENSOR AND EXHALED/INHALED AIR DETECTION METHOD**
ATEMSENSOR UND VERFAHREN ZUR ERKENNUNG VON AUSGEATMETER/INHALIERTER LUFT
CAPTEUR RESPIRATOIRE ET PROCÉDÉ DE DÉTECTION D'AIR EXPIRÉ/INHALÉ

(43) Date of publication of application: 23.10.2024
(73) Proprietor: MITSUBISHI ELECTRIC CORPORATION, Chiyoda-ku Tokyo 100-8310 (JP)
(72) Inventor: KOBAYASHI, Satoi, Tokyo 100-8310 (JP); FUJIE, Akihiro, Tokyo 100-8310 (JP); OTSUKA, Hiroshi, Tokyo 100-8310 (JP); KAMEYAMA, Shumpei, Tokyo 100-8310 (JP)
(74) Representative: Pfenning, Meinig & Partner mbB
(86) International application number: PCT/JP2022/004571
(87) International publication number: WO 2023/148946

(56) References cited:
- WO-A1-2020/152454
- WO-A1-2020/207908
- WO-A1-2021/004963
- CN-A- 111 408 081
- DE-A1- 102021 111 431
- JP-A- 2000 298 044
- JP-A- 2005 349 167
- JP-A- 2019 174 152
- US-A1- 2021 228 106
- US-A1- 2021 378 546
- US-B2- 10 520 422
- US-B2- 10 895 530

## Description

### TECHNICAL FIELD

The present disclosure relates to a respiratory sensor and an exhaled/inhaled air detection method.

### BACKGROUND ART

Conventionally, there has been disclosed a respiratory air concentration measuring sensor that includes a light emitting unit and a light receiving unit disposed in such a manner as to sandwich a respiratory air passage, and acquires gas information such as a concentration of a carbon dioxide gas in a respiratory air on the basis of a received light intensity in the light receiving unit (see Patent Literature 1).
WO 2020152454A1 discloses an infrared spectrometer having an optical circuit comprising one or more hollow waveguides provided by elongate channels formed in a substrate. The optical circuit is arranged such that infrared light in the optical circuit acquires one or more spectral properties for detection by the spectrometer. A laser source couples laser light into an input hollow waveguide portion of the optical circuit, and an optical detector receives the light from an output hollow waveguide portion of the optical circuit, allowing an analyzer to determine said one or more spectral properties from the detected light.
US 10520422 B2 discloses an optical micro-particle detector including a light source, a gas channel, and a plurality of optical detectors. The light source is configured to generate a light beam. The gas channel has at least one curved segment, which includes a light entrance and a plurality of light exits. The light beam from the light source enters the gas channel through the light entrance. The plurality of optical detectors are optically coupled to the light exits, respectively.
US 2021/0228106 A1 discloses a real-time dynamic and quantitative detection device for carbon dioxide in human exhaled air. The device comprises a face mask with an air inlet and an air outlet, an inhalation channel connected to the air inlet of the face mask, and an exhalation channel connected to the air outlet of the face mask. A first one-way valve is provided in the inhalation channel, while a second one-way valve is provided in the exhalation channel. An air path switching element is connected to the exhalation channel to switch between an exhalation air path to be detected and an inflation air path. DE 102021111431A1 discloses a monitoring system for aviation personnel, such as aircraft pilots, co-pilots, or passengers of aircraft or flight vehicles, including airplanes or helicopters used in civil or military aviation, passenger planes in scheduled or charter flights, and particularly high-speed aircraft.
WO 2020/207908 A1 discloses a system utilizing optical particle sensing. The system includes a light source, a detector for detecting the presence of light reflected back to the detector, and a processor for determining a particulate matter count or concentration based on the received light reflected by particulate matter. An optical unit along the light path suppresses direct reflections back to the detector. The detector provides a detection signal dependent on interference between the emitted light and the light reflected to the detector. In this way, an interference-based optical sensor can be reduced in size without compromising its sensing capability.

### CITATION LIST

### PATENT LITERATURE

Patent Literature 1: JP 2013-126555 A

### SUMMARY OF INVENTION

### TECHNICAL PROBLEM

The respiratory air concentration measuring sensor described in Patent Literature 1 transmits a light receiving signal from the light receiving unit to a carbon dioxide gas measuring device via a lead wire. The carbon dioxide gas measuring device detects a concentration of a carbon dioxide gas. As described above, there is a problem that it is difficult to downsize an apparatus in which the respiratory air concentration measuring sensor and the carbon dioxide gas measuring device are connected to each other by a lead wire and used.

The present disclosure has been made in order to solve the above problems, and an object of the present disclosure is to provide a respiratory sensor and an exhaled/inhaled air detection method capable of downsizing the apparatus.

### SOLUTION TO PROBLEM

The invention is defined by the appended set of claims. A respiratory sensor according to the present disclosure includes: a light wave circuit including a first light projecting unit configured to light toward exhaled air and a first light receiving unit configured to receive light emitted by the first light projecting unit, a second light projecting unit configured to emit light toward inhaled air, and a second light receiving unit configured to receive light emitted by the second light projecting unit, and formed on a semiconductor wafer; and a detection unit configured to detect a state of the exhaled air from light received by the first light receiving unit and a state of the inhaled air from light received by the second light receiving unit.

### ADVANTAGEOUS EFFECTS OF INVENTION

According to a respiratory sensor and an exhaled/inhaled air detection method of the present disclosure, an apparatus can be downsized.

### BRIEF DESCRIPTION OF DRAWINGS

FIG. 1 is a perspective view illustrating a configuration of a respiratory sensor according to a first embodiment not covered by the claims.
FIG. 2 is a block diagram illustrating a part of a configuration of the respiratory sensor according to the first embodiment.
FIG. 3 is a configuration diagram illustrating an example of a hardware configuration of a signal processing unit according to the first embodiment.
FIG. 4 is a configuration diagram illustrating an example of the hardware configuration of the signal processing unit according to the first embodiment.
FIG. 5 is a cross-sectional view illustrating a configuration of the respiratory sensor according to the first embodiment.
FIG. 6 is an enlarged diagram illustrating the configuration of the respiratory sensor according to the first embodiment.
FIG. 7 is a cross-sectional view illustrating the configuration of the respiratory sensor according to the first embodiment.
FIG. 8 is an enlarged diagram illustrating the configuration of the respiratory sensor according to the first embodiment.
FIG. 9 is a flowchart illustrating processes performed by a sensor substrate according to the first embodiment.
FIG. 10 is a block diagram illustrating a part of a configuration of a respiratory sensor according to a first modification of the first embodiment covered by the claims.
FIG. 11 is an enlarged diagram illustrating the configuration of the respiratory sensor according to the first modification of the first embodiment.
FIG. 12 is an enlarged diagram illustrating the configuration of the respiratory sensor according to the first modification of the first embodiment.
FIG. 13 is a block diagram illustrating a part of a configuration of a respiratory sensor according to a modifcation not covered by the claims of the first modification of the first embodiment.
FIG. 14 is a block diagram illustrating a part of a configuration of a respiratory sensor according to a modification covered by the claims of the first modification of the first embodiment.
FIG. 15 is an enlarged diagram illustrating the configuration of the respiratory sensor according to the first modification of the first embodiment.
FIG. 16 is a perspective view illustrating a configuration of a respiratory sensor according to the first embodiment.

### DESCRIPTION OF EMBODIMENTS

Hereinafter, embodiments according to the present disclosure will be described in detail with reference to the drawings. Note that the following embodiments illustrate specific examples. Therefore, the shape, arrangement, material, and the like of each component are merely examples, and are not intended to be limited thereto.

The drawings are schematic diagrams, and are not strictly illustrated. In the drawings, the same components are denoted by the same reference numerals.

### First Embodiment (not covered by the claims).

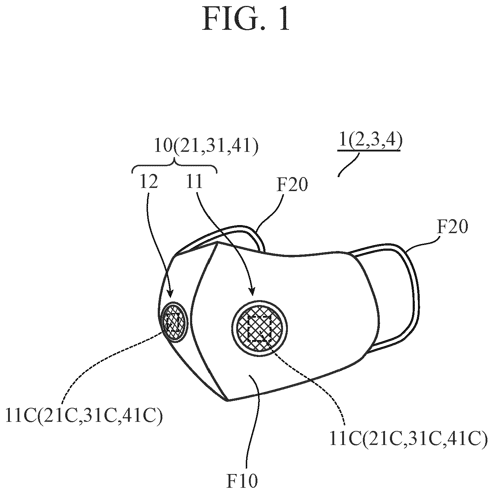

First, a configuration of a respiratory sensor 1 according to a first embodiment will be described with reference to FIG. 1. FIG. 1 is a perspective view illustrating an example of the respiratory sensor 1 according to the first embodiment in use. The respiratory sensor 1 according to the first embodiment includes a main body 10. The respiratory sensor 1 can include a plurality of the main bodies 10. The respiratory sensor 1 may include a holding member F10. The respiratory sensor 1 can be attached to the head of a human body by the holding member F10. The respiratory sensor 1 acquires information regarding exhaled air and inhaled air moving by motion of a wearer's respiratory system.

The holding member F10 holds the main body 10 around the nose or the mouth of a human body as a wearer. The holding member F10 includes, for example, ear strings F20. The ear strings F20 hold the holding member F10 on the head of the human body. The holding member F10 is made of, for example, a bendable cloth having air permeability. The holding member F10 is, for example, a mask used for hygiene or medical use. The holding member F10 is, for example, a sheet material having flexibility and air permeability, such as cloth, nonwoven fabric, or paper. Therefore, the holding member F10 is formed in such a manner as to cover the mouth and the nose of a wearer. The holding member F10 allows air to pass therethrough when the wearer breathes. The holding member F10 functions as a filter that suppresses passage of particles such as dust, pollen, and droplets. Note that the holding member only needs to be able to hold the main body 10 on a human body in such a manner that the main body 10 is located around at least one of the mouth and the nose of a human body, and for example, the holding member may be able to hold the main body 10 on the shoulders, the neck, the chest, or the like of a human body. In addition, the holding member only needs to be formed in a sheet shape while at least a part of the holding member has flexibility, and for example, a part of the holding member does not need to have a sheet shape, such as the above-described ear strings, a belt, or a hook.

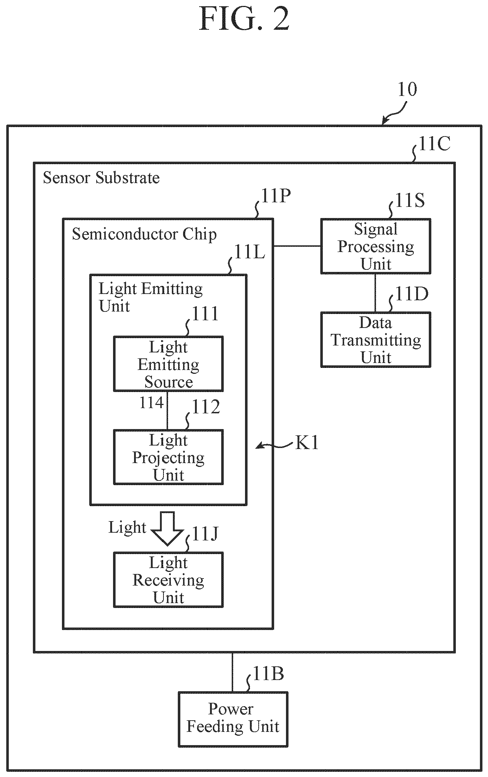

Next, a configuration of the main body 10 will be described with reference to FIG. 2. FIG. 2 is a block diagram illustrating an example of a configuration of the respiratory sensor 1 according to the first embodiment. In FIG. 2, the respiratory sensor 1 includes the main body 10. The main body 10 includes a light projecting unit 112, a light receiving unit 11J, and a signal processing unit 11S. The main body 10 may include a light emitting source 111, a data transmitting unit 11D, and a power feeding unit 11B. The light projecting unit 112 and the light receiving unit 11J are disposed on a semiconductor chip 11P. The semiconductor chip 11P is disposed, for example, on a sensor substrate 11C. The signal processing unit 11S, the light emitting source 111, and the data transmitting unit 11D may be disposed on the semiconductor chip 11P or may be disposed on the sensor substrate 11C. The power feeding unit 11B supplies power to the sensor substrate 11C. The power feeding unit 11B supplies power supplied in a non-contact manner to the sensor substrate 11C by, for example, receiving a microwave from an antenna disposed outside. The power feeding unit 11B may supply power to the sensor substrate 11C by, for example, a small battery such as a button battery. Alternatively, the power feeding unit 11B may be disposed outside the main body 10 and may supply power to the sensor substrate 11C via a power line or the like.

In FIG. 2, the sensor substrate 11C includes the semiconductor chip 11P, the signal processing unit 11S, and the data transmitting unit 11D. The sensor substrate 11C is a substrate including a conductor pattern and the like. The semiconductor chip 11P includes a light wave circuit K1 on a semiconductor wafer. In other words, the semiconductor chip 11P includes the light wave circuit K1 formed on the semiconductor wafer. The semiconductor chip 11P is formed by, for example, integrating a plurality of optical elements on a silicon substrate as a semiconductor substrate by a silicon photonics technology. The light wave circuit K1 includes, for example, a light emitting unit 11L, the light receiving unit 11J, and an optical waveguide (not illustrated). In FIG. 2, the light emitting unit 11L includes the light emitting source 111, the light projecting unit 112, and a waveguide 114.

The light emitting source 111 is a light source that emits light. The light emitting source 111 is, for example, a light emitting element.

The light emitting source 111 emits light, for example, in a predetermined wavelength band. The light emitting source 111 may be disposed outside the main body 10. The waveguide 114 transmits light. The waveguide 114 is formed as, for example, a flat light wave circuit. The light projecting unit 112 emits light. The light projecting unit 112 emits light emitted by the light emitting source 111, for example, to a space where an exhaled air or an inhaled air exists. The light receiving unit 11J receives the light emitted by the light projecting unit 112. For example, the light receiving unit 11J converts the received light into an electric signal and outputs the electric signal.

The signal processing unit 11S acquires (detects) a state of a gas from information regarding the light received by the light receiving unit 11J. The signal processing unit 11S detects, for example, particles in the gas. Specifically, the signal processing unit 11S acquires information (state) such as the types and the number of particles in the gas by detecting scattering of the light in the gas. The signal processing unit 11S detects, for example, a specific gas in the whole gas. Specifically, the signal processing unit 11S acquires information such as a concentration of the specific gas contained in the whole gas by detecting absorption of light having a specific wavelength emitted from the light projecting unit 112. The data transmitting unit 11D outputs the information to the outside. The data transmitting unit 11D transmits the information to an external device by using, for example, wireless communication. Note that the signal processing unit only needs to perform some process for detecting a state of a gas on the basis of light received by the light receiving unit. For example, the signal processing unit may acquire light received by the light receiving unit as an analog signal and perform a digital conversion process on the acquired signal, or may perform a process of outputting the acquired signal to the outside. In addition, the signal processing unit 11S constitutes a detection unit in the first embodiment.

Next, an operation of the main body 10 will be described. The light emitting source 111 generates light. The waveguide 114 transmits the light generated by the light emitting source 111 to the light projecting unit 112. The light projecting unit 112 emits the light to a space where an exhaled air or an inhaled air exists. The light emitted from the light projecting unit 112 is reflected by or passes through exhaled air or inhaled air, and is received by the light receiving unit 11J. The light receiving unit 11J converts the received light into an electric signal and outputs the electric signal to the signal processing unit 11S. The signal processing unit 11S acquires a state of exhaled air or inhaled air from information regarding the light. The data transmitting unit 11D outputs the information acquired by the signal processing unit 11S to an external device.

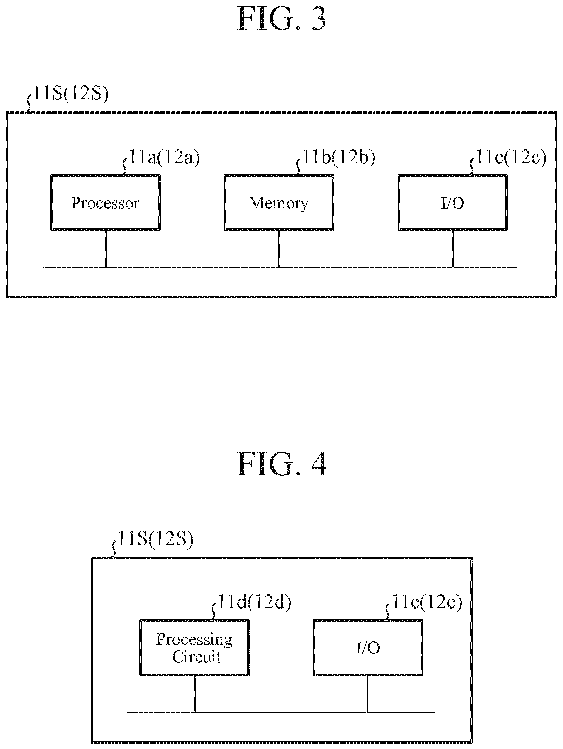

Next, a hardware configuration of the above-described signal processing unit 11S will be described with reference to FIGS. 3 and 4. FIG. 3 is a diagram illustrating an example of the hardware configuration of the signal processing unit 11S, and FIG. 4 is a diagram illustrating another example of the hardware configuration of the signal processing unit 11S. For example, as illustrated in FIG. 3, the signal processing unit 11S includes a processor 11a, a memory 11b, and an I/O port 11c, and is configured in such a manner that the processor 11a reads and executes a program stored in the memory 11b.

In addition, for example, as illustrated in FIG. 4, the signal processing unit 11S includes a processing circuit 11d as dedicated hardware and the I/O port 11c. The processing circuit 11d includes, for example, a single circuit, a composite circuit, a programmed processor, a parallel programmed processor, an application specific integrated circuit (ASIC), a field programmable gate array (FPGA), or a combination thereof. Functions of the signal processing unit 11S are implemented by the processor 11a or the processing circuit 11d as dedicated hardware executing a program.

Similarly to the signal processing unit 11S, a signal processing unit 12S may include a processor 12a, a memory 12b, and an I/O port 12c, or may include a processing circuit 12d as dedicated hardware and the I/O port 12c, and functions of the signal processing unit 12S are implemented by the processor 12a or the processing circuit 12d as dedicated hardware executing a program.

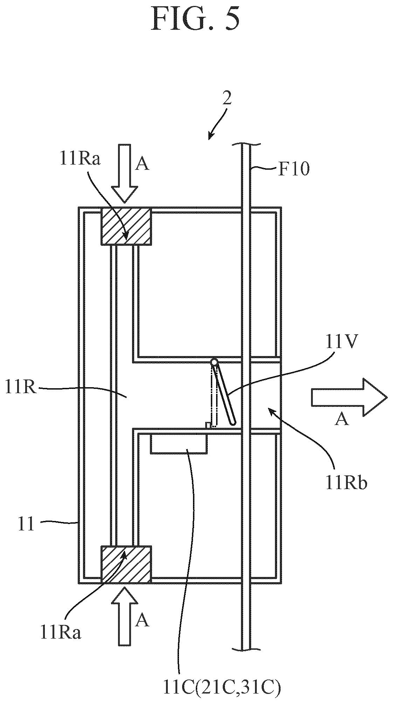

Next, an example in which a flow path of inhaled air is disposed in the main body 10 will be described. FIG. 5 is a cross-sectional view illustrating a configuration of a respiratory sensor 2 according to the first embodiment. The respiratory sensor 2 acquires information regarding inhaled air when a wearer breathes. The respiratory sensor 2 includes a main body 11. The respiratory sensor 2 may include the holding member F10. The main body 11 is held by, for example, sandwiching the holding member F10.

The main body 11 includes a sensor substrate 11C and a check valve 11V. The main body 11 may include an inhaled air guide path 11R and a power feeding unit 11B (not illustrated).

The inhaled air guide path 11R is a flow path that guides inhaled air of a wearer. The sensor substrate 11C is disposed in the inhaled air guide path 11R. The sensor substrate 11C acquires a state of inhaled air in the inhaled air guide path 11R. The light projecting unit 112 in the sensor substrate 11C emits light into the inhaled air guide path 11R. The light receiving unit 11J in the sensor substrate 11C receives light from the light projecting unit 112 via the inhaled air guide path 11R. The check valve 11V is disposed in the inhaled air guide path 11R. The check valve 11V rectifies a gas in the inhaled air guide path 11R in such a manner that the gas in the inhaled air guide path 11R flows in a wearer inhaled air direction A. The check valve 11V is, for example, a spring-type on-off valve. The check valve 11V is normally closed, but opens when a wearer sucks air. Note that the check valve 11V constitutes a second rectifying unit. The second rectifying unit only needs to rectify inhaled air in the inhaled air guide path 11R in such a manner that the inhaled air flows in a predetermined direction, and the respiratory sensor may include, instead of the check valve 11V, what rectifies a gas depending on the shape of a flow path, such as a tesla valve.

With such an arrangement, inhaled air of a wearer flows into the inhaled air guide path 11R from an upstream end 11Ra of the inhaled air guide path 11R, is guided in the inhaled air direction A through the inhaled air guide path 11R while being rectified by the check valve 11V, flows out from a downstream end 11Rb through the holding member F10, and is sent into the body of the wearer. The sensor substrate 11C is disposed upstream of the check valve 11V in the inhaled air direction A in such a manner as to face the inhaled air guide path 11R. Note that the inhaled air direction A constitutes a second direction.

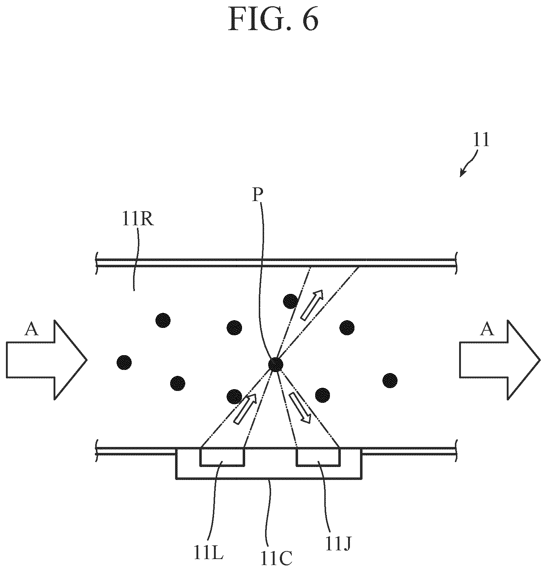

FIG. 6 is an enlarged diagram of a portion of the main body 11 according to the first embodiment where the sensor substrate 11C of the inhaled air guide path 11R is disposed. FIG. 6 is an example of a configuration capable of acquiring information regarding particles contained in inhaled air in the inhaled air guide path 11R. The light emitting unit 11L projects light toward the inhaled air guide path 11R. The light from the light emitting unit 11L is scattered by particles P in the inhaled air guide path 11R, and the scattered light is detected by the light receiving unit 11J. The signal processing unit 11S detects the particles P by acquiring information regarding the size and concentration of the particles P contained in a gas in the inhaled air guide path 11R on the basis of an electric signal from the light receiving unit 11J. For example, the signal processing unit 11S measures the size of the particles P contained in a gas in the inhaled air guide path 11R from an S/N ratio of the scattered light on the basis of the electric signal from the light receiving unit 11J. Note that, in a case where the light projecting unit 112 emits light to exhaled air, the signal processing unit 11S may be configured to acquire information regarding the size and concentration of particles contained in the exhaled air on the basis of the electric signal from the light receiving unit 11J.

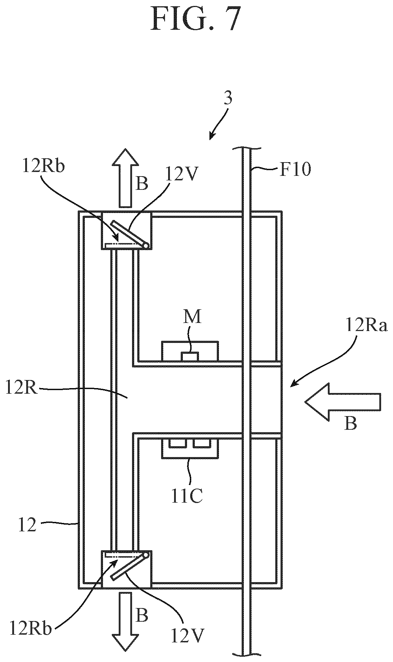

Next, an example in which a flow path of inhaled air is disposed in the main body 10 will be described. FIG. 7 is a cross-sectional view illustrating a configuration of a respiratory sensor 3 according to the first embodiment. The respiratory sensor 3 acquires information regarding exhaled air when a wearer breathes. The respiratory sensor 3 includes a main body 12. The respiratory sensor 3 may include the holding member F10. The main body 12 is held by, for example, sandwiching the holding member F10. The main body 12 includes the sensor substrate 11C and a check valve 12V. The main body 12 may include an exhaled air guide path 12R, a reflecting plate M, and the power feeding unit 11B (not illustrated).

The exhaled air guide path 12R is a flow path that guides exhaled air of a wearer. The reflecting plate M reflects light. The reflecting plate M is, for example, a mirror. The sensor substrate 11C is disposed in the exhaled air guide path 12R. The sensor substrate 11C acquires a state of exhaled air in the exhaled air guide path 12R. The light projecting unit 112 in the sensor substrate 11C emits light into the exhaled air guide path 12R. The reflecting plate M reflects light from the light projecting unit 112. The light receiving unit 11J in the sensor substrate 11C receives the light from the light projecting unit 112 via the exhaled air guide path 12R. The check valve 12V is disposed in the exhaled air guide path 12R. The check valve 12V rectifies a gas in the exhaled air guide path 12R in such a manner that the gas in the exhaled air guide path 12R flows in a wearer exhaled air direction B. The check valve 12V is, for example, a spring-type on-off valve. The check valve 12V is normally closed, but opens when a wearer exhales air. Note that the check valve 12V constitutes a first rectifying unit. The first rectifying unit only needs to rectify exhaled air in the exhaled air guide path 12R in such a manner that the exhaled air flows in a predetermined direction, and the respiratory sensor may include, instead of the check valve 12V, what rectifies a gas depending on the shape of a flow path, such as a tesla valve.

With such an arrangement, exhaled air of a wearer passes through the holding member F10 from an upstream end 12Ra of the exhaled air guide path 12R and flows into the exhaled air guide path 12R, is guided in the exhaled air direction B through the exhaled air guide path 12R while being rectified by the check valve 12V, flows out from a downstream end 12Rb, and is discharged to outside air. The sensor substrate 11C is disposed upstream of the check valve 12V in the exhaled air direction B in such a manner as to face the exhaled air guide path 12R. Note that the exhaled air direction B constitutes a first direction.

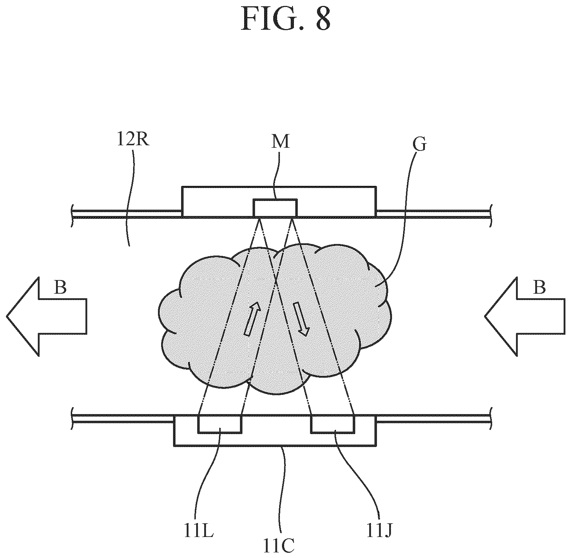

FIG. 8 is an enlarged diagram of a portion of the main body 12 according to the first embodiment where the sensor substrate 11C of the exhaled air guide path 12R is disposed. FIG. 8 is an example of a configuration capable of acquiring information regarding a component of inhaled air in the exhaled air guide path 12R. The light emitting unit 11L projects light toward the exhaled air guide path 12R. The light from the light emitting unit 11L passes through a gas in the exhaled air guide path 12R and is reflected by the reflecting plate M. The reflected light reflected by the reflecting plate M is detected by the light receiving unit 11J. The signal processing unit 11S detects a gas contained in exhaled air by acquiring information regarding a component G of a gas in the exhaled air guide path 12R on the basis of an electric signal from the light receiving unit 11J. For example, the signal processing unit 11S specifies the component G of the gas in the exhaled air guide path 12R from an absorption wavelength of the gas in the exhaled air guide path 12R on the basis of the electric signal from the light receiving unit 11J. Note that, in a case where the light projecting unit 112 emits light to inhaled air, the signal processing unit 11S may be configured to detect a gas contained in the inhaled air on the basis of the electric signal from the light receiving unit 11J.

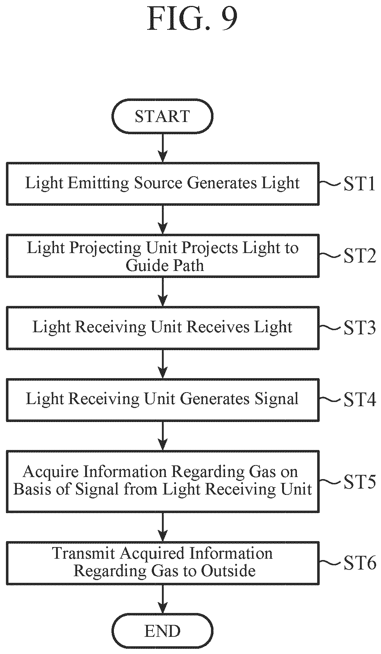

Next, processes performed by the sensor substrate 11C will be described with reference to FIG. 9. FIG. 9 is a flowchart illustrating processes performed by the sensor substrate 11C according to the first embodiment. First, the sensor substrate 11C supplies power to the light emitting source 111, and the light emitting source 111 generates light (step ST1). Next, in the sensor substrate 11C, the light projecting unit 112 projects the light generated by the light emitting source 111 toward the inhaled air guide path 11R or the exhaled air guide path 12R (step ST2). Next, in the sensor substrate 11C, the light receiving unit 11J receives the light from the light projecting unit 112 via the inhaled air guide path 11R or the exhaled air guide path 12R (step ST3), and converts the received light into an electric signal (step ST4). Next, in the sensor substrate 11C, the signal processing unit 11S acquires information regarding a gas on the basis of the electric signal output from the light receiving unit 11J (step ST5). After the process in step ST5 is performed, the acquired information regarding the gas is output from the signal processing unit 11S to the data transmitting unit 11D, and the data transmitting unit 11D transmits the information regarding the gas to the outside (step ST6). For example, the data transmitting unit 11D transmits the information regarding the gas to an external server, and the information is accumulated in the server. The respiratory sensor 1 performs exhaled/inhaled air detection through such steps, for example.

For example, in a case where information regarding the size and concentration of particles contained in inhaled air is acquired by the signal processing unit 11S, information regarding the amount of pollen or PM 2.5 contained in environment air is accumulated in a server on the basis of the size of the particles contained in the inhaled air, and a scattering situation of pollen or PM 2.5 can be found on the basis of the information accumulated in the server. In addition, for example, in a case where information regarding a component of exhaled air is acquired by the signal processing unit 11S, an oxygen saturation of a wearer can be estimated from a carbon dioxide concentration of the exhaled air of the wearer. In addition, for example, in a case where the information regarding a component of exhaled air is acquired by the signal processing unit 11S, a blood alcohol concentration of a wearer can be estimated from an alcohol concentration of the exhaled air of the wearer. That is, the "state of exhaled air or inhaled air" in the present disclosure indicates a state such as the sizes and number of particles contained in a gas or a concentration of alcohol or carbon dioxide contained in the gas.

The respiratory sensor 1, 2, 3 may include a plurality of the main bodies 10, 11, 12. For example, the respiratory sensor 2 can detect two types of particles by including two main bodies 11. In this case, the two main bodies 11 are adjusted in such a manner as to detect particles having different sizes. For example, the respiratory sensor 3 can detect two types of gases by including two main bodies 12. In this case, the two main bodies 12 are adjusted in such a manner as to detect different gases. Furthermore, the respiratory sensor 2 can simultaneously acquire information regarding exhaled air and information regarding inhaled air by including, for example, the main body 11 and the main body 12. In addition, the main body 11 and the main body 12 may be configured to acquire the same information. Both the main body 11 and the main body 12 may be configured to acquire information regarding a concentration of carbon dioxide, and may be configured to estimate the amount of oxygen consumed in the body of a wearer by comparing an oxygen concentration of inhaled air with an oxygen concentration of exhaled air.

As described above, the main body 10 according to the first embodiment can be downsized and reduced in weight by including the light wave circuit on the semiconductor wafer. The main body 10 can downsize an apparatus that acquires information regarding a gas by using a Si photosensor chip, for example, as compared with a case where an optical element is disposed on a printed wiring board. As a result, for example, the main body 10 can be used for various applications such as being attachable to a flexible mask or the like. A plurality of the main bodies 10 can be held even by the lightweight holding member F10 such as a mask.

In addition, the respiratory sensor 1 can include the main body 11 and the main body 12. As a result, for example, information regarding inhaled air of a wearer and information regarding exhaled air of the wearer can be simultaneously acquired. Furthermore, by acquiring exhaled air and inhaled air in association with each other and comparing the exhaled air and the inhaled air with each other, information regarding a change in the inhaled air in a living body can be acquired, and a state such as a physical condition or a fatigue level of the wearer can be estimated.

In addition, the main body 10 can select exhaled air or inhaled air by using the check valve 11V, 12V. Note that the signal processing unit 11S may be configured to determine whether a gas to be detected is exhaled air or inhaled air from a state of the gas based on a detection result. In such a case, the respiratory sensor does not need to use the check valve 11V, 12V. In this case, the respiratory sensor can acquire states of exhaled air and inhaled air only by including one main body 10.

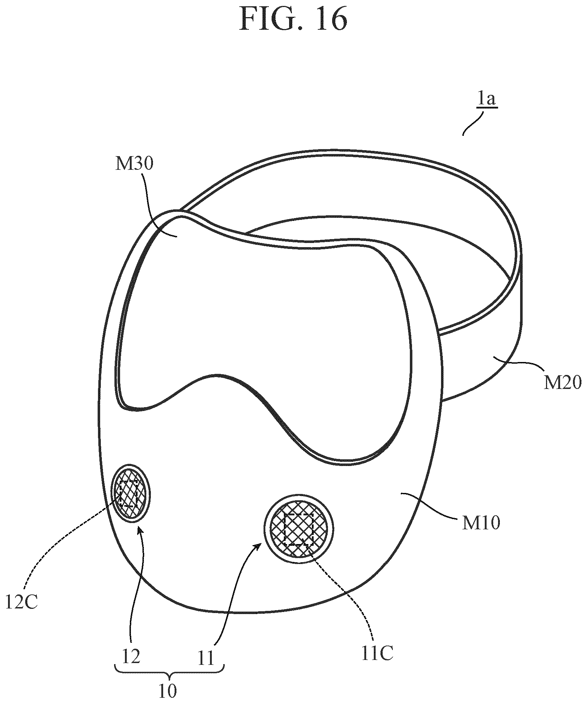

The holding member F10 in FIG. 1 has a mask shape, but may have a shape other than the mask shape. FIG. 16 is a perspective view illustrating an example of a respiratory sensor 1a according to the first embodiment in use. FIG. 16 illustrates the respiratory sensor 1a including a holding member M10 having a shape covering a face. The respiratory sensor 1a is different from the respiratory sensor 1 of FIG. 1 in a range covering a head of a human body and the holding member M10 held on the head, but other components are similar to those of the respiratory sensor 1 of FIG. 1, and thus redundant description will be omitted. The holding member M10 covers the nose, the mouth, and the both eyes of a wearer. The holding member M10 includes a belt M20 to be held on a head of a wearer and a light transmitting portion M30 having a light transmitting property at a portion corresponding to the positions of both eyes of the wearer. Note that the holding member M10 may be formed in, for example, a helmet shape covering the entire head above a neck of a wearer.

### Modification 1 (covered by the claims).

Next, Modification 1 of the first embodiment will be described. In Modification 1, the main body 10 acquires a state of a gas at two places by including a plurality of the light projecting units 112. At this time, by emitting light having different wavelengths from the light projecting units 112, information regarding different gases can be acquired. A respiratory sensor 4 according to Modification 1 of the first embodiment has a similar configuration to that in FIG. 1, and thus the description thereof will be omitted. Note that the respiratory sensor 4 includes a main body other than the main body 10.

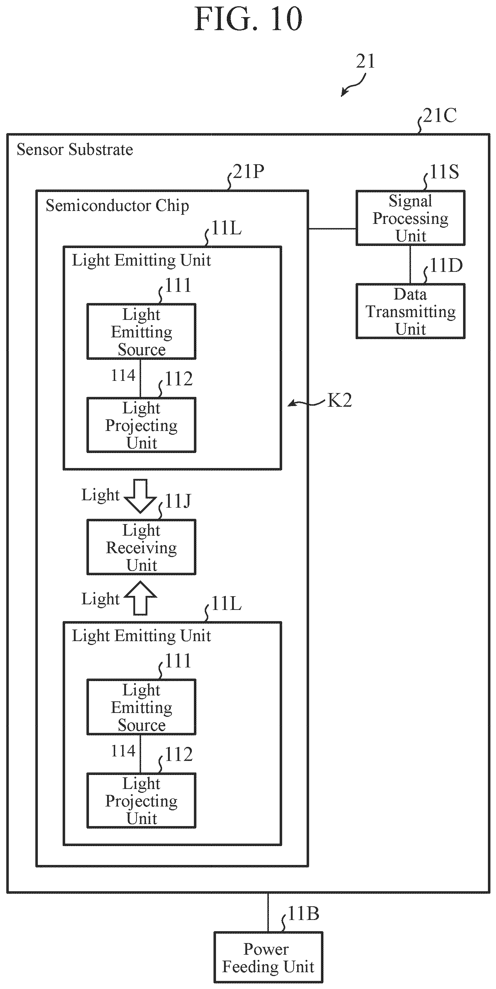

Next, a case where the respiratory sensor 4 includes a main body 21 will be described. FIG. 10 is a block diagram illustrating a part of a configuration of the main body 21 according to Modification 1 of the first embodiment. The main body 21 is different from the main body 10 of FIG. 2 in including two light emitting units 11L, but is similar to the main body 10 of FIG. 2 in the other points. Similarly to the main body 11, 12, the main body 21 may include the inhaled air guide path 11R, the exhaled air guide path 12R, the check valve 11V, 12V, the reflecting plate M, and the like.

A semiconductor chip 21P includes two light emitting units 11L and one light receiving unit 11J. Wavelength bands of light emitted from the two light emitting units 11L may be different from each other. The light receiving unit 11J receives the light from the two light emitting units 11L.

A sensor substrate 21C includes the semiconductor chip 21P. The sensor substrate 21C may include the signal processing unit 11S and the data transmitting unit 11D. Note that the two light projecting units 112 and 112 included in the two light emitting units 11L and 11L constitute a first light projecting unit and a second light projecting unit, respectively. As described above, the semiconductor chip 21P according to Modification 1 includes a light wave circuit K2 including the light projecting units 112 and the light receiving unit 11J and formed on a semiconductor wafer.

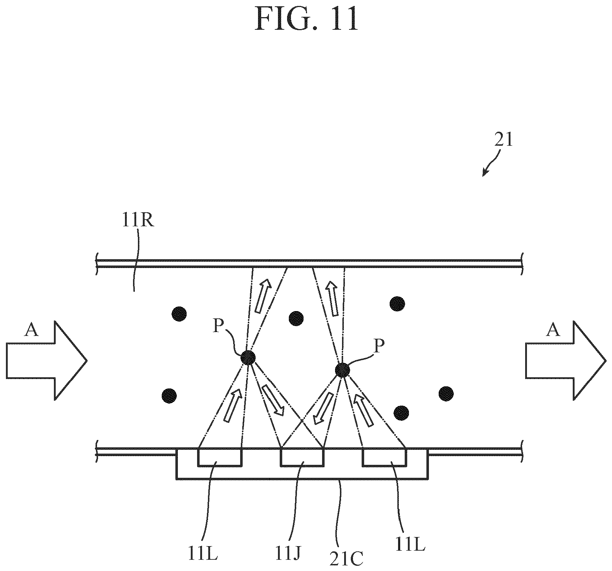

FIG. 11 is an enlarged diagram illustrating the main body 21 according to Modification 1 of the first embodiment. In the main body 21, the two light emitting units 11L project light toward the inhaled air guide path 11R. The light receiving unit 11J receives the light projected by the two light emitting units 11L via the inhaled air guide path 11R. In the main body 21, the light receiving unit 11J receives the light from the two light emitting units 11L, converts the light into an electric signal, and outputs the electric signal. The signal processing unit 11S acquires information regarding a gas in the inhaled air guide path 11R on the basis of the electric signal output from the light receiving unit 11J. Since information regarding the gas at two places is acquired, more accurate information can be acquired than that in a case where there is one light emitting unit.

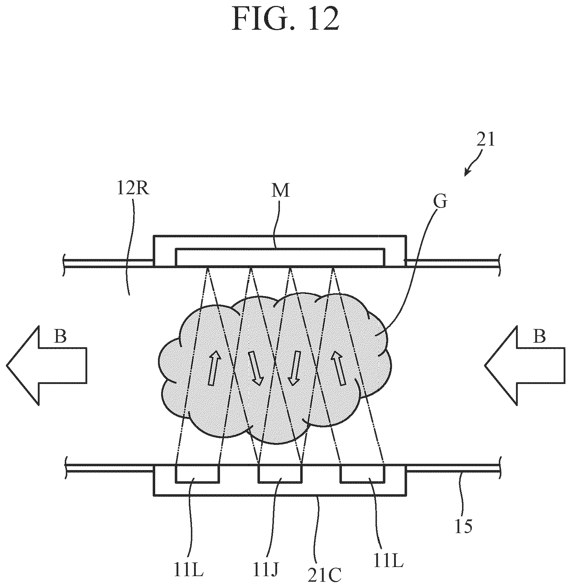

FIG. 12 is an enlarged diagram of another example illustrating the main body 21 according to Modification 1 of the first embodiment. In the main body 21, the two light emitting units 11L project light toward the exhaled air guide path 12R. The light receiving unit 11J receives the light projected by the two light emitting units 11L via the exhaled air guide path 12R. In the main body 21, the light receiving unit 11J receives the light from the two light emitting units 11L, converts the light into an electric signal, and outputs the electric signal, and the signal processing unit 12S acquires information regarding a gas in the exhaled air guide path 12R on the basis of the electric signal output by the light receiving unit 11J. Since information regarding the gas at two places is acquired, more accurate information can be acquired than that in a case where there is one light emitting unit.

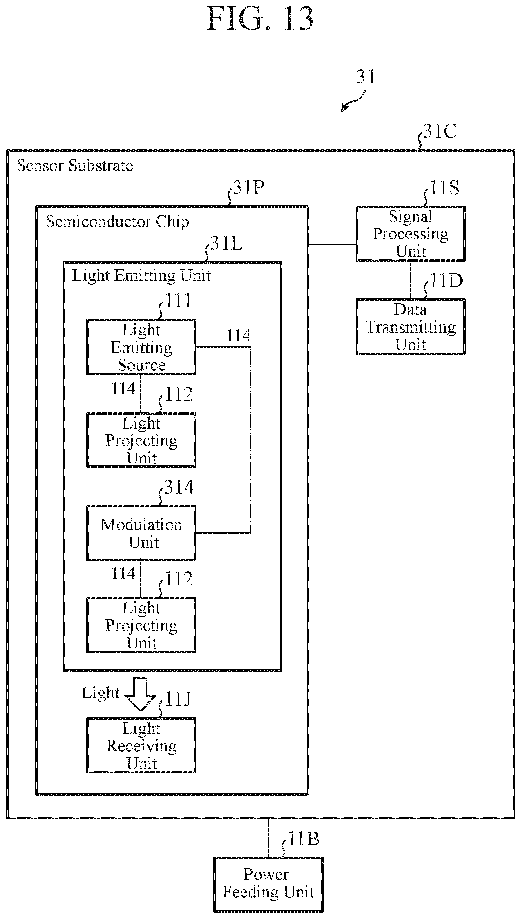

Next, a case where the respiratory sensor 4 includes a main body 31 will be described. FIG. 13 is a block diagram illustrating a part of a configuration of the main body 31 according a modification not covered by the claims of Modification 1 of the first embodiment. The main body 31 is different from the main body 10 of FIG. 2 in including two light projecting units 112 and a modulation unit 314, but is similar to the main body 10 of FIG. 2 in the other points. Similarly to the main body 11, 12, the main body 31 may include the inhaled air guide path 11R, the exhaled air guide path 12R, the check valve 11V, 12V, the reflecting plate M, and the like. The main body 31 includes a sensor substrate 31C. The main body 31 may include the power feeding unit 11B that supplies power to the sensor substrate 31C.

A semiconductor chip 31P of the sensor substrate 31C includes a light emitting unit 31L and the light receiving unit 11J. The light emitting unit 31L includes one light emitting source 111 and two light projecting units 112. The light emitting source 111 and the light projecting unit 112 are connected to each other by the waveguide 114. Alternatively, the light emitting source 111 and the modulation unit 314 are connected to each other by the waveguide 114, and the modulation unit 314 and the light projecting unit 112 are connected to each other by the waveguide 114. The modulation unit 314 modulates a wavelength of light. Therefore, wavelengths of light emitted from the two light projecting units 112 are different from each other.

In the main body 31, light having different wavelengths is projected from the two light projecting units 112 to the inhaled air guide path 11R, and received by the light receiving unit 11J. Therefore, for example, a plurality of components having different absorption wavelengths contained in the gas can be detected.

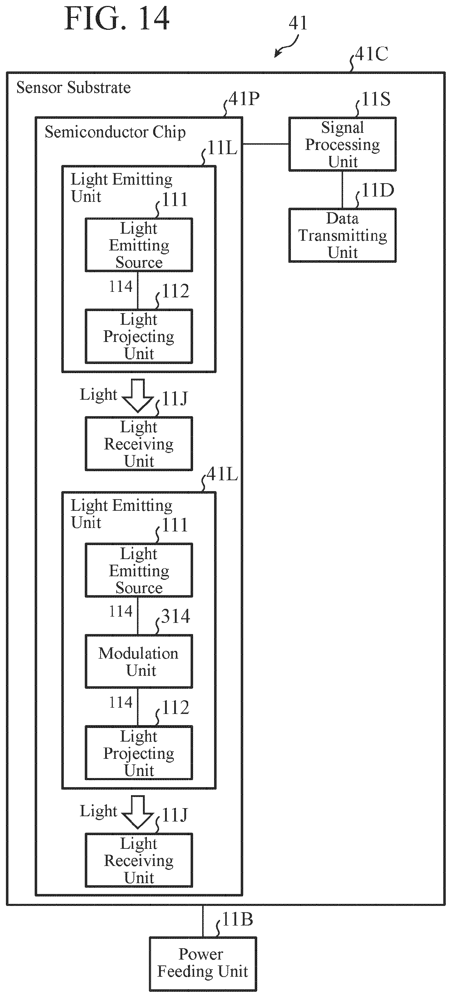

Next, a case where the respiratory sensor 4 includes a main body 41 will be described. FIG. 14 is a block diagram illustrating a modification covered by the claims of Modification 1 of the first embodiment. The main body 41 is different from the main body 10 of FIG. 2 in including light emitting units 11L and 41L, two light receiving units 11J, and the modulation unit 314. The main body 41 is the same as the main body 10 of FIG. 2 in the other points. Similarly to the main body 11, 12, the main body 41 may include the inhaled air guide path 11R, the exhaled air guide path 12R, the check valve 11V, 12V, the reflecting plate M, and the like. The main body 41 includes a sensor substrate 41C. The main body 41 may include the power feeding unit 11B that supplies power to the sensor substrate 41C.

A semiconductor chip 41P of the sensor substrate 41C includes the light emitting units 11L and 41L and the two light receiving units 11J. The light emitting unit 41L includes the light emitting source 111, the modulation unit 314, and the light projecting unit 112. In the light emitting unit 41L, the light emitting source 111 and the modulation unit 314 are connected to each other by the waveguide 114, and the modulation unit 314 and the light projecting unit 112 are connected to each other by the waveguide 114. The modulation unit 314 modulates a wavelength of light. Therefore, wavelengths of light emitted from the two light projecting units 112 are different from each other. The two light receiving units 11J receive light from respective different light projecting units 112. Note that the two light projecting units 112 and 112 constitute a first light projecting unit and a second light projecting unit, respectively, and the two light receiving units 11J and 11J constitute a first light receiving unit and a second light receiving unit, respectively.

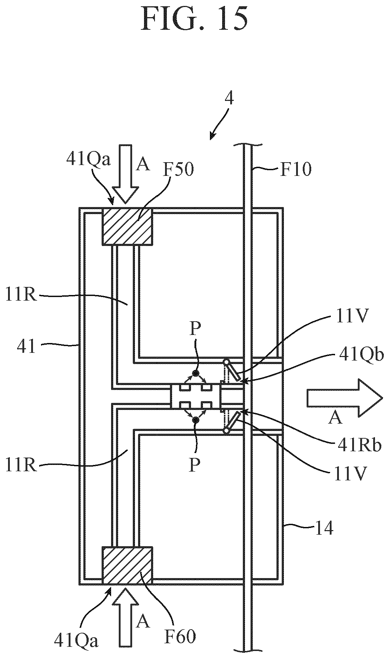

FIG. 15 is a cross-sectional view illustrating a configuration of the respiratory sensor 4 according to Modification 1 of the first embodiment. The respiratory sensor 4 includes the main body 41. The respiratory sensor 4 may include the holding member F10, the inhaled air guide path 11R, the exhaled air guide path 12R, the check valve 11V, 12V, the reflecting plate M, a filter F50, F60, and the like. The filter F50, F60 prevents entry of particles in air. In FIG. 15, the main body 41 forms the two inhaled air guide paths 11R independent of each other. The sensor substrate 41C is disposed between the two inhaled air guide paths 11R, and acquires information regarding a gas in the two inhaled air guide paths 11R. The filter F50 or the filter F60 is disposed at an upstream end 41Qa of the inhaled air guide path 11R. The filter F50 and the filter F60 have different roughness. The check valves 11V are disposed at each of downstream ends 41Qb and 41Rb of the inhaled air guide path 11R.

Each of the two light receiving units 11J (see FIG. 14) in the sensor substrate 41C receives light projected by either of the two light projecting units 112 (see FIG. 14) in the sensor substrate 41C via the inhaled air guide path 11R, converts the received light into an electric signal, and outputs the electric signal. In the main body 41, the filter F50, F60 causes only particles having sizes equal to or less than a specific size to pass therethrough to the downstream end 41Qb, 41Rb of the inhaled air guide path 11R, light having a wavelength corresponding to the size of each of the particles is projected, and information regarding a gas can be thereby acquired.

As described above, the respiratory sensor 4 can emit light having a plurality of different wavelengths by including the plurality of light projecting units 112 in the main body 41. As a result, the respiratory sensor 4 can detect a plurality of different types of gases. In addition, the respiratory sensor 4 makes it easy for the two light receiving units 11J to detect particles having different sizes by limiting the sizes of particles entering the main body 41 using the filter F50, F60. The respiratory sensor 4 can acquire a state of exhaled air or inhaled air by including the light projecting unit 112 and the light receiving unit 11J as one set, and therefore can acquire a plurality of states of the exhaled air or the inhaled air by including a plurality of the sets.

Note that, in the above-described embodiment and modification, the light emitting unit and the light receiving unit are not limited to those formed as a silicon photosensor chip, and may be those formed as a non-silicon photosensor. In addition, in the above-described embodiment and modification, the signal processing unit, the data transmitting unit, and the power feeding unit may be disposed on a single semiconductor chip, may be disposed on different semiconductor chips independent of each other, or may be disposed on different substrates independent of each other.

Note that, in the present disclosure, an embodiment and modifications thereof can be freely combined to each other, any constituent element in the embodiment and the modifications thereof can be modified, or any constituent element in the embodiment and the modifications thereof can be omitted. However, the invention is defined by the claims.

### INDUSTRIAL APPLICABILITY

The respiratory sensor according to the present disclosure can be used for acquiring information regarding a component of a gas or particles contained in the gas.

### REFERENCE SIGNS LIST

1, 1a, 2, 3, 4: respiratory sensor, 10, 11, 12, 21, 31, 41: main body, 11B: power feeding unit, 11C, 21C, 31C, 41C: sensor substrate, 11D: data transmitting unit, 11J: light receiving unit, 11L, 31L, 41L: light emitting unit, 11P, 21P, 31P, 41P: semiconductor chip, 11R: inhaled air guide path, 11Ra: upstream end, 11Rb: downstream end, 11S, 12S: detection unit (signal processing unit), 11V, 12V: check valve, 11a, 12a: processor, 11b, 12b: memory, 11c, 12c: I/O port, 11d, 12d: processing circuit, 12R: exhaled air guide path, 12Ra: upstream end, 12Rb: downstream end, 41Qa: upstream end, 41Qb: downstream end, 41Rb: downstream end, 111: light emitting source, 112: light projecting unit, 114: waveguide, 314: modulation unit, A: second direction (inhaled air direction), B: first direction (exhaled air direction), F10: holding member, F20: ear string, F50, F60: filter, G: component, K1, K2: light wave circuit, M: reflecting plate, M10: holding member, M20: belt, M30: light transmitting portion, P: particle

## Claims

1. A respiratory sensor (1, 1a, 2, 3, 4) comprising:
a light wave circuit (K1, K2) including a first light projecting unit (112) configured to emit light toward exhaled air, a first light receiving unit (11J) configured to receive light emitted by the first light projecting unit, a second light projecting unit (112) configured to emit light toward inhaled air, and a second light receiving unit (11J) configured to receive light emitted from the second light projecting unit, and formed on a semiconductor wafer; and
a detection unit (11S, 12S) configured to detect a state of the exhaled air from light received by the first light receiving unit and a state of the inhaled air from light received by second light receiving unit,
wherein the respiratory sensor further comprises:
an exhaled air guide path (12R) through which the exhaled air is guided; and
an inhaled air guide path (11R) through which the inhaled air is guided, wherein
the first light projecting unit is configured to emit light to the exhaled air in the exhaled air guide path, and
the second light projection unit is configured to emit light to the inhaled air in the inhaled air guide path.

2. The respiratory sensor according to claim 1, further comprising
a holding member (F10) capable of holding the light wave circuit on a human body in such a manner that the light wave circuit is located around a mouth and a nose of the human body.

3. The respiratory sensor according to claim 2, wherein
the holding member has flexibility and is formed in a sheet shape.

4. The respiratory sensor according to any one of claims 1 to 3, wherein
the detection unit is configured to detect a particle (P) contained in the exhaled air or the inhaled air.

5. The respiratory sensor according to any one of claims 1 to 3, wherein
the detection unit is configured to detect a gas contained in the exhaled air on a basis of absorption of light having a specific wavelength emitted from the first light projecting unit.

6. The respiratory sensor according to claim 1, wherein
the first light projecting unit is configured to emit light having a plurality of wavelengths.

7. The respiratory sensor according to claim 1, further comprising:
a first rectifying unit (12V) to rectify the exhaled air in such a manner that the exhaled air in the exhaled air guide path flows in a first direction; and
a second rectifying unit (11V) to rectify the inhaled air in such a manner that the inhaled air in the inhaled air guide path flows in a second direction.

8. The respiratory sensor according to claim 1, wherein
the detection unit is configured to determine whether a detection target is exhaled air or inhaled air on a basis of a detection result.

9. An exhaled/inhaled air detection method performed by the respiratory sensor (1, 1a, 2, 3, 4) according to any one of claims 1 to 8, the respiratory sensor including: a light wave circuit (K1, K2) including a first light projecting unit (112), a first light receiving unit (11J), a second light projecting unit (112), and a second light receiving unit (11J), and formed on a semiconductor wafer; and a detection unit (11S, 12S), the exhaled/inhaled air detection method comprising:
emitting, by the first light projecting unit, light toward exhaled air;
receiving, by the first light receiving unit, light emitted by the first light projecting unit;
emitting, by the second light projecting unit, light toward inhaled air;
receiving, by the second light receiving unit, light emitted by the second light projecting unit; and
detecting, by the detection unit, a state of the exhaled air from light received by the first light receiving unit and a state of the inhaled air from light received by the second light receiving unit.

## Patentansprüche

1. Atmungssensor (1, 1a, 2, 3, 4), umfassend:
eine Lichtwellenschaltung (K1, K2), die auf einem Halbleiterwafer ausgebildet ist und die eine erste Lichtprojektionseinheit (112), die eingerichtet ist, Licht in Richtung Ausatemluft zu emittieren, eine erste Lichtempfangseinheit (11J), die eingerichtet ist, von der ersten Lichtprojektionseinheit emittiertes Licht zu empfangen, eine zweite Lichtprojektionseinheit (112), die eingerichtet ist, Licht in Richtung Einatemluft zu emittieren, und eine zweite Lichtempfangseinheit (11J) aufweist, die eingerichtet ist, von der zweiten Lichtprojektionseinheit emittiertes Licht zu empfangen, und
eine Erfassungseinheit (11S, 12S), die eingerichtet ist, einen Zustand der Ausatemluft aus dem von der ersten Lichtempfangseinheit empfangenen Licht und einen Zustand der Einatemluft aus dem von der zweiten Lichtempfangseinheit empfangenen Licht zu erfassen,
wobei der Atmungssensor ferner umfasst:
einen Ausatemluftführungsweg (12R), durch den die Ausatemluft geführt wird; und
einen Einatemluftführungsweg (11R), durch den die Einatemluft geführt wird,
wobei
die erste Lichtprojektionseinheit eingerichtet ist, Licht in die Ausatemluft im Ausatemluftführungsweg zu emittieren, und
die zweite Lichtprojektionseinheit eingerichtet ist, Licht in die Einatemluft im Einatemluftführungsweg zu emittieren.

2. Atmungssensor nach Anspruch 1, ferner umfassend
ein Halteelement (F10), das in der Lage ist, die Lichtwellenschaltung an einem menschlichen Körper so zu halten, dass die Lichtwellenschaltung um einen Mund und eine Nase des menschlichen Körpers herum angeordnet ist.

3. Atmungssensor nach Anspruch 2, wobei
das Halteelement Flexibilität aufweist und in flächenartiger Form ausgebildet ist.

4. Atmungssensor nach einem der Ansprüche 1 bis 3, wobei
die Erfassungseinheit eingerichtet ist, ein in der Ausatemluft oder der Einatemluft enthaltenes Partikel (P) zu erfassen.

5. Atmungssensor nach einem der Ansprüche 1 bis 3, wobei
die Erfassungseinheit ein in der Ausatemluft enthaltenes Gas auf Grundlage von Absorption von Licht, aufweisend eine spezifische Wellenlänge, das von der ersten Lichtprojektionseinheit emittiert wird, zu erfassen.

6. Atmungssensor nach Anspruch 1, wobei
die erste Lichtprojektionseinheit eingerichtet ist, ein Licht zu emittieren, das eine Vielzahl von Wellenlängen aufweist.

7. Atmungssensor nach Anspruch 1, ferner umfassend:
eine erste Gleichausrichtungseinrichtung (12V), um die Ausatemluft so gleich auszurichten, dass die Ausatemluft im Ausatemluftführungsweg in eine erste Richtung strömt; und
eine zweite Gleichausrichtungseinrichtung (11V), um die Einatemluft so gleich auszurichten, dass die Einatemluft im Einatemluftführungsweg in eine zweite Richtung strömt.

8. Atmungssensor nach Anspruch 1, wobei
die Erfassungseinheit eingerichtet ist, auf der Grundlage eines Erfassungsergebnisses zu bestimmen, ob es sich bei einem Erfassungsziel um Ausatemluft oder Einatemluft handelt.

9. Ausatem-/Einatemluft-Erfassungsverfahren, das von dem Atmungssensor (1, 1a, 2, 3, 4) nach einem der Ansprüche 1 bis 8 durchgeführt wird, wobei der Atmungssensor aufweist: eine Lichtwellenschaltung (K1, K2), die auf einem Halbleiterwafer ausgebildet ist und eine erste Lichtprojektionseinheit (112), eine erste Lichtempfangseinheit (11J), eine zweite Lichtprojektionseinheit (112) und eine zweite Lichtempfangseinheit (11J) aufweist; und eine Erfassungseinheit (11S, 12S), wobei das Ausatem-/Einatemluft-Erfassungsverfahren umfasst:
Emittieren, durch die erste Lichtprojektionseinheit, von Licht in Richtung der Ausatemluft;
Empfangen, durch die erste Lichtempfangseinheit, von Licht, das von der ersten Lichtprojektionseinheit emittiert wird;
Emittieren, durch die zweite Lichtprojektionseinheit, von Licht in Richtung der Einatemluft;
Empfangen, durch die zweite Lichtempfangseinheit, von Licht, das von der zweiten Lichtprojektionseinheit emittiert wird; und
Erfassen, durch die Erfassungseinheit, eines Zustands der Ausatemluft aus dem von der ersten Lichtempfangseinheit empfangenen Licht und eines Zustands der Einatemluft aus dem von der zweiten Lichtempfangseinheit empfangenen Licht.

## Revendications

1. Capteur respiratoire (1, 1a, 2, 3, 4) comprenant :
un circuit d'onde lumineuse (K1, K2) incluant une première unité de projection de lumière (112) configurée pour émettre de la lumière vers de l'air expiré, une première unité de réception de lumière (11J) configurée pour recevoir de la lumière émise par la première unité de projection de lumière, une seconde unité de projection de lumière (112) configurée pour émettre de la lumière vers de l'air inhalé, une seconde unité de réception de lumière (11J) configurée pour recevoir de la lumière émise depuis la seconde unité de projection de lumière, et formé sur une tranche de semi-conducteur ; et
une unité de détection (115, 12S) configurée pour détecter un état de l'air expiré à partir de lumière reçue par la première unité de réception de lumière et un état de l'air inhalé à partir de lumière reçue par seconde unité de réception de lumière,
dans lequel le capteur respiratoire comprend en outre :
un chemin de guidage d'air expiré (12R) à travers lequel l'air expiré est guidé ; et
un chemin de guidage d'air inhalé (11R) à travers lequel l'air inhalé est guidé,
dans lequel
la première unité de projection de lumière est configurée pour émettre de la lumière vers l'air expiré dans le chemin de guidage d'air expiré, et
la seconde unité de projection de lumière est configurée pour émettre de la lumière vers l'air inhalé dans le chemin de guidage d'air inhalé.

2. Capteur respiratoire selon la revendication 1, comprenant en outre
un élément de maintien (F10) capable de maintenir le circuit d'onde lumineuse sur un corps humain d'une manière telle que le circuit d'onde lumineuse est situé autour d'une bouche et d'un nez du corps humain.

3. Capteur respiratoire selon la revendication 2, dans lequel
l'élément de maintien présente une flexibilité et est formé dans une forme de feuille.

4. Capteur respiratoire selon l'une quelconque des revendications 1 à 3, dans lequel
l'unité de détection est configurée pour détecter une particule (P) contenue dans l'air expiré ou l'air inhalé.

5. Capteur respiratoire selon l'une quelconque des revendications 1 à 3, dans lequel
l'unité de détection est configurée pour détecter un gaz contenu dans l'air expiré sur une base d'absorption de lumière ayant une longueur d'onde spécifique émise depuis la première unité de projection de lumière.

6. Capteur respiratoire selon la revendication 1, dans lequel
la première unité de projection de lumière est configurée pour émettre de la lumière ayant une pluralité de longueurs d'onde.

7. Capteur respiratoire selon la revendication 1, comprenant en outre :
une première unité de rectification (12V) pour rectifier l'air expiré d'une manière telle que l'air expiré dans le chemin de guidage d'air expiré s'écoule dans une première direction ; et
une seconde unité de rectification (11V) pour rectifier l'air inhalé d'une manière telle que l'air inhalé dans le chemin de guidage d'air inhalé s'écoule dans une seconde direction.

8. Capteur respiratoire selon la revendication 1, dans lequel
l'unité de détection est configurée pour déterminer si une cible de détection est de l'air expiré ou de l'air inhalé sur une base d'un résultat de détection.

9. Procédé de détection d'air expiré/inhalé mis en œuvre par le capteur respiratoire (1, 1a, 2, 3, 4) selon l'une quelconque des revendications 1 à 8, le capteur respiratoire incluant : un circuit d'onde lumineuse (K1, K2) incluant une première unité de projection de lumière (112), une première unité de réception de lumière (11J), une seconde unité de projection de lumière (112), et une seconde unité de réception de lumière (11J), et formé sur une tranche de semi-conducteur; et une unité de détection (115, 125), le procédé de détection d'air expiré/inhalé comprenant :
l'émission, par la première unité de projection de lumière, de lumière vers de l'air expiré ;
la réception, par la première unité de réception de lumière, de lumière émise par la première unité de projection de lumière ;
l'émission, par la seconde unité de projection de lumière, de lumière vers de l'air inhalé ;
la réception, par la seconde unité de réception de lumière, de lumière émise par la seconde unité de projection de lumière ; et
la détection, par l'unité de détection, d'un état de l'air expiré à partir de la lumière reçue par la première unité de réception de lumière et d'un état de l'air inhalé à partir de lumière reçue par la seconde unité de réception de lumière.
